# EUROPEAN PATENT SPECIFICATION

(11) **EP 2 823 842 B1**
(45) Date of publication and mention of the grant of the patent: **13.04.2016**
(21) Application number: 13194973.7
(22) Date of filing: 28.11.2013
(51) Int. Cl.: A61M 5/31, A61M 39/20, A61M 39/10

(54) **Integrated syringe having anti-releasing-type open-and-shut cap structure**
Integrierte Spritze mit Öffnungs- und Verschlusskappenstruktur mit Freisetzungsschutz
Seringue intégrée ayant une structure de capuchon d'ouverture/fermeture de type anti-libération

(30) Priority: 11.07.2013 KR 20130081412
(43) Date of publication of application: 14.01.2015
(73) Proprietor: Cho, Hee Min, Gunsan-city, Jeollabuk-do (KR)
(72) Inventor: Cho, Hee Min, Gunsan-city, Jeollabuk-do (KR)
(74) Representative: Spengler, Robert

(56) References cited:
- WO-A1-2013/047042
- KR-A- 20010 113 219
- KR-B1- 101 027 861

## Description

### BACKGROUND OF THE INVENTION

### 1. Field of the Invention

The present invention relates to an integrated syringe using an open-and-shut cap to seal a luer-lock that is fastened to an outer circumference of a sleeve with a reduced diameter so as to fasten a needle to an upper end of a syringe body and is connected to other devices and, more particularly, to an integrated syringe having an anti-releasing-type open-and-shut cap structure, in which, when an open-and-shut cap is fastened -to a luer-lock in a threaded fastening method, a locking groove and a locking protrusion of a tension locker correspondingly formed on the open-and-shut cap and the luer-lock, respectively, perform a locking function, so that a natural release of the open-and-shut cap is prevented as long as an external force is exerted, thus guaranteeing seal safety, effectively preventing a loss of injection fluid due to leakage and preventing the penetration of germs, and in which the luer-lock is integrally coupled (fused or welded) to an upper portion of a syringe body, thus preventing the syringe body from being contaminated by the injection fluid, and in addition, preventing a neck of a needle from being broken due to an integrated structure.

### 2. Description of the Related Art

Document KR 101 027 861 B1 discloses an integrated syringe having an anti-releasing-type open-and-shut cap structure, the syringe including a luer-lock inserted into a sleeve of a syringe body, and an open-and-shut cap having a fastening portion fastened to a fastening hole of the luer-lock in a threaded fastening manner. A locking groove is formed on the fastening portion of the luer-lock.

As is generally known, a syringe is an important medical device and is used for injecting a medical fluid into a body, for collecting blood, or for other medical purposes.

Such a syringe includes a syringe body made of synthetic resin or glass material, a piston inserted into a rear of the syringe body, a needle coupled to a sleeve on an upper portion of the syringe body as necessary, an open-and-shut cap, other blood collecting devices, etc.

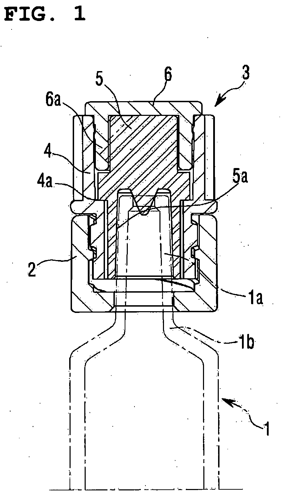

Here, it may be difficult to directly couple the needle, the open-and-shut cap and other blood collecting devices to the sleeve on the upper portion of the syringe body. In order to address the problem, according to known embodiments, a luer-lock 2 is integrally provided on a sleeve 1a on the upper portion of a syringe body 1 or is assembled with the sleeve 1a as shown in FIG. 1, thus allowing the needle, the open-and-shut cap, other blood collecting devices, etc. to be coupled to the luer-lock 2.

In particular, the open-and-shut cap 3 serves to seal the syringe body 1 when one desires to store, carry and transport the syringe body 1.

Such an open-and-shut cap 3 is configured such that a packing 5 with an airtight sealing groove 5a to which the sleeve 1a is coupled air-tight is inserted into a rear of a stepped hole 4a in a body 4 and is closed by a "T"-shaped cover 6, with an insert portion 6a of the "T"-shaped cover 6 being inserted into a space between the body 4 and the packing 5.

Such an open-and-shut cap 3 is fastened to a fastening hole of the luer-lock 2 in the threaded fastening method, thus allowing the syringe body 1 to be used in a sealed state.

However, a conventional syringe has the following problems.

First, the syringe is problematic in that the open-and-shut cap 3 is simply fastened to the luer-lock 2 coupled to the sleeve 1a of the syringe body 1 in the threaded fastening method, and has no locking means when the fastening has been completed, so that the threaded fastening structure of the open-and-shut cap may be easily released if the syringe is subjected to external force, shock or shaking.

That is, the threaded fastening of the open-and-shut cap 3 is naturally released, so that the seal safety for the syringe becomes poor.

In addition, as described above, the natural release of the open-and-shut cap 3 leads to the leakage of a medical fluid from the syringe body 1 and thus the contamination of its surrounding. Consequently, a loss of injection fluid that is to be injected into a patient, the penetration of germs into the injection fluid, and several other fatal problems may occur.

Further, since the open-and-shut cap 3 is coupled simply by inserting the insert portion 6a of the "T"-shaped cover 6 between the body 4 and the packing 5, the coupling force is weak. Thus, when pressure in the syringe body acts on the packing 5, the "T"-shaped cover may be easily dislodged from a predetermined position between the body 4 and the packing 5.

That is, defects in open-and-shut cap 3 frequently occur.

Second, the syringe is problematic in that the luer-lock 2 is coupled to the sleeve 1a provided on an end of the syringe body 1 by insertion, so that a strong coupling force is not obtained between the luer-lock 2 and the sleeve 1a of the syringe body 1, unlike an integrated structure.

That is, when the luer-lock 2 is fastened to the open-and-shut cap 3 in the threaded fastening method or is separated from the open-and-shut cap 3, undesirable movement or torsion may occur in the sleeve 1a or a torsion load may be concentrated on the neck portion 1b between the syringe body 1 and the sleeve 1a, so that the neck portion 1b may be easily broken.

Moreover, since a spacing gap is formed in a fastening surface between the luer-lock 2 and the sleeve 1a of the syringe body 1, the injection fluid may flow down to the syringe body 1 through the spacing gap when the leakage of the injection fluid occurs, thus contaminating the syringe body 1 and its surroundings.

The foregoing is intended merely to aid in the understanding of the background of the present invention, and is not intended to mean that the present invention falls within the purview of the related art that is already known to those skilled in the art.

### SUMMARY OF THE INVENTION

Accordingly, the present invention has been made keeping in mind the above problems occurring in the related art, and an object of the present invention is to provide an integrated syringe, in which, when an open-and-shut cap is fastened to a luer-lock in a threaded fastening method, a locking groove and a locking protrusion of a tension locker correspondingly formed on the open-and-shut cap and the luer-lock, respectively, perform a locking function, so that a natural release of the open-and-shut cap is prevented, thus guaranteeing excellent seal safety, effectively preventing the leakage of injection fluid and a loss of the injection fluid and preventing the penetration of germs.

Another object of the present invention is to provide an integrated syringe, in which a luer-lock is integrally coupled (fused or welded) to an upper portion of a syringe body, thus preventing the syringe body from being contaminated by injection fluid, and in addition, the undesirable movement and torsion of the luer-lock are prevented when the open-and-shut cap is coupled in a threaded fastening method or is separated, thus preventing a connecting portion between the syringe body and the sleeve provided on an upper portion thereof, namely, the neck portion, from being broken.

In order to accomplish the above objects, the present invention provides an integrated syringe having an anti-releasing-type open-and-shut cap structure, the syringe including a luer-lock inserted into a sleeve of a syringe body, and an open-and-shut cap having a fastening portion fastened to a fastening hole of the luer-lock in a threaded fastening manner, wherein a locking groove is formed on the fastening portion of the open-and-shut cap, and a tension locker having a locking protrusion to be inserted into the locking groove of the open-and-shut cap is formed on the fastening hole of the luer-lock in such a way as to provide tension inwards and outwards, thus preventing a natural release due to a locking operation when the open-and-shut cap is fastened in the threaded fastening manner.

Each of the locking groove of the open-and-shut cap and the locking protrusion of the tension locker of the luer-lock may comprise at least two along an outer circumference in such a way that the locking groove and the locking protrusion correspond to each other.

The tension locker may be configured to provide tension in a space formed along a circumference of the luer-lock in such a way as to pass from an inside to an outside thereof, the tension locker having an inclined surface that is reduced in thickness towards an end where the locking protrusion is formed, thus enhancing a larger tension force.

The open-and-shut cap may be configured such that a packing is coupled to a rear of a stepped hole in a body and is closed by a "T"-shaped cover, and an insert portion of the "T"-shaped cover is inserted between a space between the body and the packing, and a holding portion formed along an outer circumference of the "T"-shaped cover is coupled to surround and support an outer circumference of the body.

The luer-lock may have on a lower portion thereof a ring-shaped groove along a contact portion that is in close contact with an end surface of the syringe body, so that the luer-lock and the syringe body may be integrally coupled to each other by fusing via a medium filled in the groove.

According to the present invention, in the state where the lower surface of the luer-lock comes into close contact with the end surface of the syringe body, the luer-lock and the syringe body may be integrally coupled to each other by heat welding.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a sectional view showing important parts of a conventional syringe;
FIG. 2 is a perspective view showing the coupled state of a luer-lock with an open-and-shut cap according to the present invention;
FIG. 3 is an exploded perspective view of FIG. 2;
FIG. 4 is an enlarged perspective view showing important parts of the luer-lock according to the present invention;
FIG. 5 is a front sectional view showing the configuration of FIG. 2;
FIG. 6 is an enlarged view showing portion A encircled in FIG. 5;
FIG. 7 is an enlarged view showing portion B encircled in FIG. 5;
FIG. 8 is a plan sectional view showing the configuration of FIG. 2;
FIG. 9 is a perspective view showing the coupled state of a luer-lock with an open-and-shut cap according to another embodiment of the present invention; and
FIG. 10 is a sectional view showing the configuration in an installed state of FIG. 9.

### DESCRIPTION OF PREFERRED EMBODIMENTS

Hereinafter, preferred embodiments of the present invention will be described in detail with reference to the accompanying drawings.

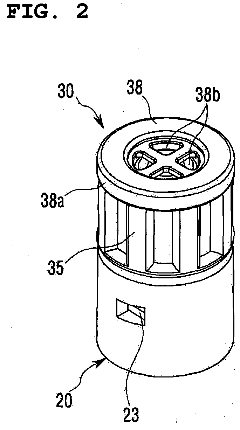

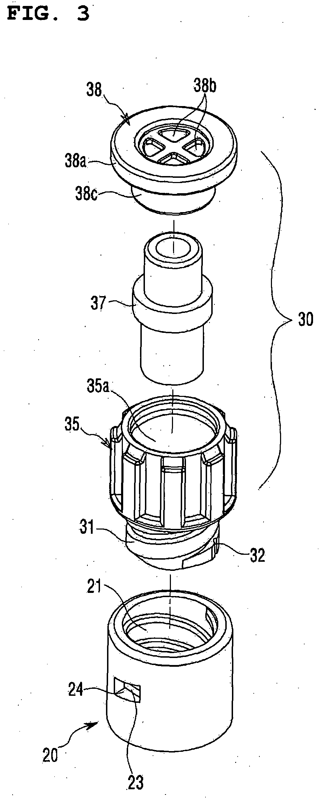

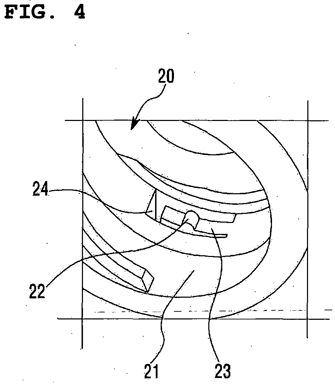

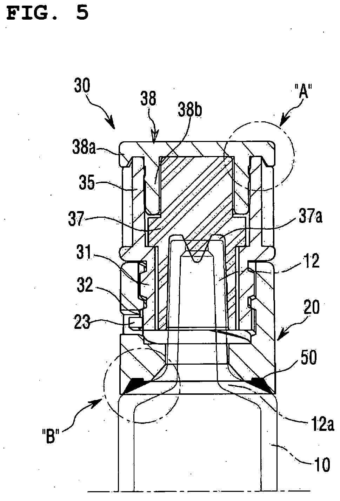

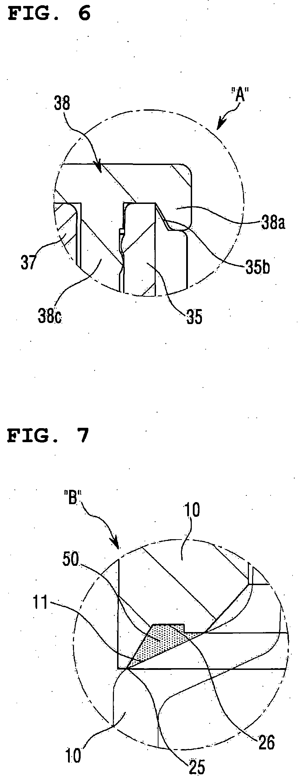

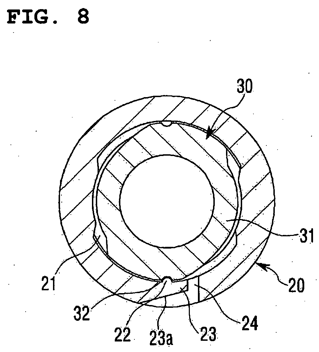

As shown in FIGS. 2 to 10, an integrated syringe having an anti-releasing-type open-and-shut cap structure according to the present invention includes a luer-lock 20 that is inserted into a sleeve 12 of a syringe body 10, and an open-and-shut cap 30 having a fastening portion 31 that is fastened to a fastening hole 21 of the luer-lock 20 in a threaded fastening manner.

Particularly, a locking groove 32 is formed on the fastening portion 31 of the open-and-shut cap 30, while a tension locker 23 having a locking protrusion 22 to be inserted into the locking groove 32 of the open-and-shut cap 30 is formed on the fastening hole 21 of the luer-lock 20 in such a way as to provide tension inward and outward, thus preventing a natural release due to a locking operation when the threaded fastening for the open-and-shut cap 30 has been completed.

In this respect, each of the locking groove 32 of the open-and-shut cap 30 and the locking protrusion 22 of the tension locker 23 of the luer-lock 20 may be one or at least two along an outer circumference in such a way that the locking groove 32 and the locking protrusion 22 correspond to each other.

The tension locker 23 is configured such that it provides tension in a space 24 that is formed along a circumference of the luer-lock 20 in such a way as to pass from an inside to an outside thereof, and has an inclined surface 23a which is reduced in thickness towards an end where the locking protrusion 22 is formed, thus ensuring a larger tension force.

Further, the open-and-shut cap 30 is configured such that a packing 37 is coupled to a rear of a stepped hole 35a in a body 35 and is closed by a "T"-shaped cover 38. An insert portion 38b of the "T"-shaped cover 38 is inserted between a space between the body 35 and the packing 37, and a holding portion 38a formed along an outer circumference of the "T"-shaped cover 38 is coupled to surround and support an outer circumference of the body 35.

In this respect, an insert stepped groove 35b is formed on the outer circumference of the body 35 such that the holding portion 38a of the "T"-shaped closure cover 38 is inserted therein.

Furthermore, a gas hole 38b is formed in an outer portion of the "T"-shaped cover 38 to allow sterilization gas to be injected in a sterilization operation.

Moreover, the luer-lock 20 has on a lower portion thereof a ring-shaped groove 26 along a contact portion 25 that is in close contact with an end surface 11 of the syringe body 10. Thereby, the luer-lock 20 made of a resin material, such as PE, PP, PC material, and the syringe body 10 made of a glass material are integrally coupled to each other via a medium 50 filled in the groove 26.

Here, the medium 50 utilizes a glass glue solution. As a fusing method, a thermal fusing method using heat, an electric-beam fusing method using electric beams, or an UV fusing method using ultraviolet rays is utilized. However, although not shown in the drawings, it is possible to couple the luer-lock 20 with the syringe body 10 by directly heating the luer-lock 20 and the end surface 11 of the syringe body 10 without using the medium. Of course, the luer-lock 20 and the syringe body 10 may be integrally coupled to each other by various other coupling means.

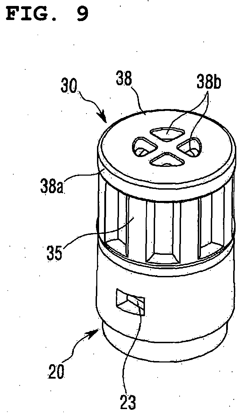

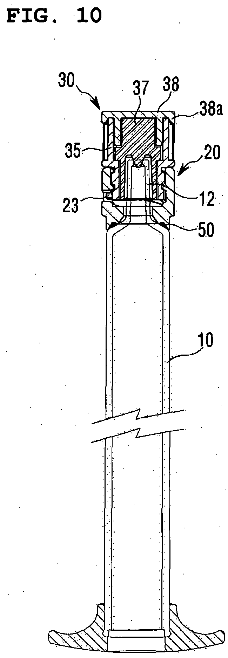

Further, the luer-lock 20 is formed in a cylindrical shape having the same diameter as that of the syringe body 10, depending on the volume of the syringe body 10, for example, on a difference in diameter corresponding to volumes such as 5mℓ, 3mℓ, etc. Alternatively, as shown in FIGS. 9 and 10, in order to conform to the diameter of the syringe body 10, the luer-lock 20 is formed in a stepped shape along the outer circumference thereof.

Reference numeral 37a denotes an airtight sealing groove of the packing 37 to which the sleeve 12 is coupled air-tight.

The operation and effects of the present invention configured as described above will be described in the following.

The syringe of the present invention is assembled by inserting the luer-lock 20 into the sleeve 12 of the syringe body 10.

In this respect, the contact portion 25 provided on the lower portion of the luer-lock 20 comes into close contact with the end surface 11 of the syringe body 10, and the ring-shaped groove 26 formed along the contact portion 25 is filled with the medium 50 such as the glass glue solution.

Further, by the thermal fusing method of applying heat to the medium 50, the electric-beam fusing method of applying electric beams to the medium 50, or the UV fusing method of applying UV to the medium 50, the luer-lock 20 and the syringe body 10 are integrally fused to each other.

Moreover, of course, by directly heating the luer-lock 20 and the end surface 11 of the syringe body 10 without using the medium 50, the luer-lock 20 and the syringe body 10 may be fused and coupled to a single structure.

As such, the open-and-shut cap 30 is fastened, in the threaded fastening manner, to the luer-lock 20 that is integrally coupled to the sleeve 12 of the syringe body 10, thus enabling the syringe body 10 to be used in the sealed state.

In this respect, when the fastening portion 31 of the open-and-shut cap 30 and the fastening hole 21 of the luer-lock 20 are fastened to each other in the threaded fastening manner, the locking protrusion 22 of the tension locker 23 provided on the fastening hole 21 of the luer-lock 20 is inserted into the locking groove 32 formed on the fastening portion 31 of the open-and-shut cap 30, thus performing the locking function.

In particular, the tension locker 23 is configured to provide tension in the space 24 that is formed along the circumference of the luer-lock 20 in such a way as to pass from the inside to the outside thereof, and has the inclined surface 23a which is reduced in thickness towards the' end where the locking protrusion 22 is formed, thus ensuring a larger tension force inwards or outwards.

That is, the open-and-shut cap 30 and the luer-lock 20 perform the locking function while they are fastened to each other in the threaded fastening manner by the locking groove 32 and the locking protrusion 22 of the tension locker 23, thus preventing the open-and-shut cap 30 from being naturally released.

Therefore, when the open-and-shut cap 30 is fastened to the luer-lock 20 in the threaded fastening manner, the locking groove 32 and the locking protrusion 22 of the tension locker 23 perform the locking operation. For this reason, as long as external force is not applied repeatedly, the natural release of the open-and-shut cap 30 is prevented. Consequently, excellent seal safety is guaranteed, and the leakage of injection fluid from the syringe body 10 is prevented, so that contamination and a loss of injection fluid are prevented, and the penetration of germs into the injection fluid is perfectly prevented.

Moreover, the luer-lock 20 is integrally coupled (welded or fused) to the end surface 11 of the syringe body 10, thus preventing the syringe body 10 and its surrounding from being contaminated by the injection fluid. The syringe body 10 and the luer-lock 20 are integrally coupled to each other without exposing a neck portion 12a of the sleeve 12, thus fundamentally solving a problem of the prior art wherein the neck portion 12a is broken by torsion stress when the syringe body 10 and the luer-lock 20 are coupled to or separated from each other.

Further, the open-and-shut cap 30 of the present invention is configured such that the packing 37 is coupled to the rear of the stepped hole 35a in the body 35 and is closed by the "T"-shaped cover 38. The insert portion 38b of the "T"-shaped cover 38 is inserted between the space between the body 35 and the packing 37, and the holding portion 38a formed along the outer circumference of the "T"-shaped cover 38 is coupled to surround and doubly support the outer circumference of the body 35.

Therefore, when the open-and-shut cap 30 is fastened to the luer-lock 20 coupled to the upper portion of the syringe body 10, a clamping force resulting from the threaded fastening operation is generated. Thereby, although pressure acting on the end of the sleeve 12 pushes the packing 37 upwards, the "T"-shaped closure cover 38 outside the packing 37 doubly supports both the inner and outer positions of the body 35 by the holding portion 38a and the insert portion 38b, thus providing a strong coupling force. As a result, the present invention can fundamentally solve the problem of the prior art wherein the "T"-shaped closure cover 38 is separated from the body 35.

As described above, the present invention provides an integrated syringe, in which, when an open-and-shut cap is fastened to a luer-lock in a threaded fastening method, a locking groove and a locking protrusion of a tension locker correspondingly formed on the open-and-shut cap and the luer-lock, respectively, perform a locking function, so that the natural release of the open-and-shut cap is prevented as long as an artificial force is not applied, thus guaranteeing excellent seal safety, effectively preventing the leakage of injection fluid and a loss of the injection fluid and preventing the penetration of germs, and in which the luer-lock is integrally coupled (fused or welded) to an end of a syringe body, thus preventing the syringe body and its surrounding from being contaminated by the leaking injection fluid, and in addition, preventing a neck portion of the syringe body from being broken.

Further, the present invention provides an integrated syringe, in which a "T"-shaped cover of an open-and-shut cap is improved in coupling structure, so that a strong coupling force for the "T"-shaped cover is maintained when pressure in a syringe body acts on a packing, thus keeping the assembled state of the "T"-shaped cover excellent.

That is, the integrated syringe of the present invention has been remarkably improved through improvements in techniques of the product, thus achieving both convenience and safety in use and thereby contributing to technical advancement for medical devices.

Although a preferred embodiment of the present invention has been described for illustrative purposes, those skilled in the art will appreciate that various modifications, additions and substitutions are possible, without departing from the scope of the invention as disclosed in the accompanying claims.

## Claims

1. An integrated syringe having an anti-releasing-type open-and-shut cap structure, the syringe including a luer-lock (20) inserted into a sleeve (12) of a syringe body (10), and an open-and-shut cap (30) having a fastening portion (31) fastened to a fastening hole (21) of the luer-lock (20) in a threaded fastening manner,
**characterized in that**
a locking groove (32) is formed on the fastening portion (31) of the open-and-shut cap (30), and a tension locker (23) having a locking protrusion (22) to be inserted into the locking groove (32) of the open-and-shut cap (30) is formed on the fastening hole (21) of the luer-lock (20) in such a way as to provide tension inwards and outwards, thus preventing a natural release due to a locking operation when the open-and-shut cap (30) is fastened in the threaded fastening manner.

2. The integrated syringe as set forth in claim 1, wherein each of the locking groove (32) of the open-and-shut cap (30) and the locking protrusion (22) of the tension locker (23) of the luer-lock (20) comprises one pair at two opposite points along an outer circumference in such a way that the locking groove (32) and the locking protrusion (22) correspond to each other.

3. The integrated syringe as set forth in claim 1 or 2, wherein the tension locker (23) is configured to provide tension in a space (24) formed along a circumference of the luer-lock (20) in such a way as to pass from an inside to an outside thereof, the tension locker (23) having an inclined surface (23a) that is reduced in thickness towards an end where the locking protrusion (22) is formed, thus enhancing a tension force.

4. The integrated syringe as set forth in claim 1, wherein the open-and-shut cap (30) is configured such that a packing (37) is coupled to a rear of a stepped hole (35a) in a body (35) and is closed by a "T"-shaped cover (38), and an insert portion (38b) of the "T"-shaped cover (38) is inserted between a space between the body (35) and the packing (37), and a holding portion (38a) formed along an outer circumference of the "T"-shaped cover (38) is coupled to surround and support an outer circumference of the body (35).

5. The integrated syringe as set forth in claim 1, wherein the luer-lock (20) has on a lower portion thereof a ring-shaped groove (26) along a contact portion (25) that is in close contact with an end surface (11) of the syringe body (10), so that the luer-lock (20) and the syringe body (10) are integrally coupled to each other by fusing via a medium (50) filled in the groove (26).

## Patentansprüche

1. Kombinierte Injektionsspritze mit einer Kappenstruktur zum Öffnen und Schließen der lösungssicheren Art, wobei die Injektionsspritze einen Luer-Lock (20) beinhaltet, der in eine Hülse (12) eines Spritzenkörpers (10) eingesetzt ist, und eine Kappe zum Öffnen und Schließen (30) mit einem Befestigungsabschnitt (31), der mittels Verschraubung an einer Befestigungsöffnung (21) des Luer-Locks (20) befestigt ist,
**dadurch gekennzeichnet, dass**
am Befestigungsabschnitt (31) der Kappe zum Öffnen und Schließen (30) eine Arretierungsrille (32) gebildet ist und eine Spannungsarretierung (23) mit einem Arretierungsvorsprung (22), der in die Arretierungsrille (32) der Kappe zum Öffnen und Schließen (30) einzusetzen ist, derart an der Befestigungsöffnung (21) des Luer-Locks (20) gebildet ist, dass eine Spannung nach innen und außen bereitgestellt ist und somit infolge eines Arretierungsvorgangs ein natürliches Lösen verhindert wird, wenn die Kappe zum Öffnen und Schließen (30) mittels Verschraubung befestigt ist.

2. Kombinierte Injektionsspritze nach Anspruch 1, wobei die Arretierungsrille (32) der Kappe zum Öffnen und Schließen (30) und der Arretierungsvorsprung (22) der Spannungsarretierung (23) des Luer-Locks (20) an zwei entgegengesetzten Punkten entlang eines Außenumfangs derart ein Paar bilden, dass die Arretierungsrille (32) und der Arretierungsvorsprung (22) einander entsprechen.

3. Kombinierte Injektionsspritze nach Anspruch 1 oder 2, wobei die Spannungsarretierung (23) dafür gestaltet ist, in einem Raum (24), der entlang eines Umfanges des Luer-Locks (20) gebildet ist, derart eine Spannung bereitzustellen, dass diese von innen nach außen verläuft, wobei die Spannungsarretierung (23) eine schräge Oberfläche (23a) aufweist, deren Dicke sich zu dem Ende hin verringert, an dem der Arretierungsvorsprung (22) gebildet ist, was eine Spannkraft verstärkt.

4. Kombinierte Injektionsspritze nach Anspruch 1, wobei die Kappe zum Öffnen und Schließen (30) derart gestaltet ist, dass eine Packung (37) an eine Hinterseite einer abgestuften Öffnung (35a) in einem Körper (35) gekoppelt und durch eine T-förmige Abdeckung (38) verschlossen ist, und ein Einsatzabschnitt (38b) der T-förmigen Abdeckung (38) zwischen einem Raum zwischen dem Körper (35) und der Packung (37) eingesetzt ist und ein Halteabschnitt (38a), der entlang eines Außenumfangs der T-förmigen Abdeckung (38) gebildet ist, so gekoppelt ist, dass er einen Außenumfang des Körpers (35) umgibt und abstützt.

5. Kombinierte Injektionsspritze nach Anspruch 1, wobei der Luer-Lock (20) an seinem unteren Abschnitt eine ringförmige Rille (26) entlang eines Kontaktabschnitts (25) aufweist, der in engem Kontakt mit einer Endfläche (11) des Spritzenkörpers (10) steht, so dass der Luer-Lock (20) und der Spritzenkörper (10) durch Verschmelzen mittels eines Mediums (50), das in die Rille (26) gefüllt ist, miteinander kombiniert gekoppelt sind.

## Revendications

1. Seringue intégrée ayant une structure de capuchon d'ouverture et de fermeture de type anti-libération, la seringue comprenant un raccord Luer (20) inséré dans un manchon (12) d'un corps de seringue (10), et un capuchon d'ouverture et de fermeture (30) ayant une partie de fixation (31) fixée à un trou de fixation (21) du raccord Luer (20) par filetage,
**caractérisée en ce que** :
une rainure de verrouillage (32) est formée sur la partie de fixation (31) du capuchon d'ouverture et de fermeture (30), et un dispositif de verrouillage de tension (23) ayant une saillie de verrouillage (22) à insérer dans la rainure de verrouillage (32) du capuchon d'ouverture et de fermeture (30) est formé sur le trou de fixation (21) du raccord Luer (20) afin de fournir la tension vers l'intérieur et vers l'extérieur, empêchant ainsi une libération naturelle due à une opération de verrouillage, lorsque le capuchon d'ouverture et de fermeture (30) est fixé par filetage.

2. Seringue intégrée selon la revendication 1, dans laquelle chacune parmi la rainure de verrouillage (32) du capuchon d'ouverture et de fermeture (30) et la saillie de verrouillage (22) du dispositif de verrouillage de tension (23) du raccord Luer (20) comprend une paire, à deux points opposés le long d'une circonférence externe, de sorte que la rainure de verrouillage (32) et la saillie de verrouillage (22) correspondent entre elles.

3. Seringue intégrée selon la revendication 1 ou 2, dans laquelle le dispositif de verrouillage de tension (23) est configuré pour fournir la tension dans un espace (24) formé le long d'une circonférence du raccord Luer (20) afin de passer de son intérieur à son extérieur, le dispositif de verrouillage de tension (23) ayant une surface inclinée (23a) qui est réduite en épaisseur vers une extrémité où la saillie de verrouillage (22) est formée, améliorant ainsi une force de tension.

4. Seringue intégrée selon la revendication 1, dans laquelle le capuchon d'ouverture et de fermeture (30) est configuré de sorte qu'une garniture (37) est couplée à une partie arrière d'un trou étagé (35a) dans un corps (35) et est fermé par un couvercle en forme « T » (38), et une partie d'insert (38b) du couvercle en forme de « T » (38) est insérée entre un espace entre le corps (35) et la garniture (37), et une partie de maintien (38a) formée le long d'une circonférence externe du couvercle en forme de « T » (38) est couplée pour entourer et supporter une circonférence externe du corps (35).

5. Seringue intégrée selon la revendication 1, dans laquelle le raccord Luer (20) a, sur sa partie inférieure, une rainure de forme annulaire (26) le long d'une partie de contact (25) qui est en contact immédiat avec une surface d'extrémité (11) du corps de seringue (10), de sorte que le raccord Luer (20) et le corps de seringue (10) sont couplés de manière solidaire entre eux par fusion via un milieu (50) versé dans la rainure (26).
